# EUROPEAN PATENT APPLICATION

(11) **EP 2 009 423 A1**
(43) Date of publication of application: **31.12.2008**
(21) Application number: 07252627.0
(22) Date of filing: 28.06.2007
(51) Int. Cl.: G01N 1/40, G01N 33/26, G01N 31/02

(54) **Sample plate**

(71) Applicant: BP OIL INTERNATIONAL LIMITED, Sunbury-on-Thames TW16 7BP (GB)
(72) Inventor: The designation of the inventor has not yet been filed
(74) Representative: De Kezel, Eric

(57) **Abstract**

A sample plate for a portable analysis apparatus for analysis of a solid precipitated from a fluid sample, which sample plate comprises a sample inlet, a precipitation zone, a filter and an analysis zone, the sample plate being adapted to allow:
(i) a fluid sample to be fed through the sample inlet into the precipitation zone;
(ii) a precipitant to be fed to the precipitation zone;
(iii) conditions within the precipitation zone to be maintained such that precipitation occurs when the fluid sample and precipitant mix to form a suspension;
(iv) separation of the solid in the suspension by the filter;
(v) addition of a solvent to the filter to dissolve the solid and form a solution; and
(vi) analysis of the solution in the analysis zone.

## Description

This invention relates to analytical apparatus, more specifically to a portable apparatus and a method for determining the quantity of a precipitate obtained from a liquid.

There are numerous sources of crude oil, with often widely varying chemical and physical properties. For example, crude oils such as West Texas Intermediate have a high proportion of relatively low boiling point hydrocarbon components, whereas Venezuelan crude oils for example tend to comprise a higher proportion of components with higher boiling points. Due to the wide variation in crude oil types, it is advantageous for an oil refinery to be able to use as wide a variety of crude oil feedstocks as possible in order to reduce reliance on any single crude oil source, and also to allow the use of low cost feedstocks when available.

Different crude oils often behave very differently within the refinery, and hence their impact on refining operations and process equipment will also vary. Therefore, analysis of a crude oil that either has been procured or is to be procured is important in establishing its suitability for use, and the likely extent of any negative impact on the refinery processes and/or equipment.

Descriptions of refinery processes, and products derived therefrom, are well-known to the person skilled in the art, and are described, for example, under the chapter entitled "Oil Refining", by Walther W. Irion and Otto S. Neuwirth, in Ullmann's Encyclopedia of Industrial Chemistry, published by Wiley.

Crude oil is typically analysed for properties such as boiling point range, acidity/basicity, asphaltenes content, sulphur content and aromatics content. This can be achieved by transporting a sample of crude oil to a laboratory equipped with appropriate analytical facilities. On-line measurements can also be made, where suitable on-line analysers are available, and where it is possible to make such measurements. Known on-line analytical techniques include optical absorption and/or spectroscopic techniques.

However, it is not uncommon for feedstocks to be purchased while crude oil is still in transit, for example in a crude oil tanker. Without proper analytical data, a purchaser of a crude oil cargo must make a number of assumptions on the value of the feedstock in order to determine whether or not to make a trade. It would be advantageous if analytical data of a cargo could be made rapidly, so that the results can be made available to the potential purchaser in a timely manner.

The rapid analysis of products of refinery processes is also desirable. Such products include intermediates in the overall refinery process, end products such as fuels, lubricants or bitumen, process streams from the overall refinery process which are subsequently used as chemical feedstocks, for example naphtha which can be fed to a steam cracker to produce olefins, and products from the overall refinery process which are subsequently used as fuels or lubricants, or as blending components for fuels or lubricants, as well as the fuels (e.g. aviation fuel, gasoline, diesel and marine fuels) and lubricants themselves. The rapid analysis of formulated products, fuels and lubricants is also desirable, for example at a terminal, at a pipeline, in the distribution system, or at a point of sale.

According to the present invention, there is provided a sample plate for a portable analytical apparatus for the analysis of a solid precipitated from a fluid sample, which sample plate comprises a sample inlet, a precipitation zone, a filter and an analysis zone, the sample plate being adapted to allow:
(i) a fluid sample to be fed through the inlet into the precipitation zone;
(ii) a precipitant to be fed to the precipitation zone;
(iii) conditions within the precipitation zone to be maintained such that precipitation occurs when the fluid sample and precipitant mix to form a suspension;
(iv) separation of the solid in the suspension by the filter;
(v) addition of a solvent to the filter to dissolve the solid and form a solution; and
(vi) analysis of the solution in the analysis zone.

According to a second aspect of the present invention, the apparatus can be used for analysing a precipitate derived from a sample fluid.

The sample plate of the present invention can be combined with a base portion to form a portable analysis device. The portable analysis device is designed to be readily transportable so that analysis is not restricted to laboratory or manufacturing site locations. For example, it can be easily transported to a remote storage tank, a ship cargo, a rail wagon or a road tanker, for example, in order to analyse the fluid contents therein.

The sample plate can be used for the analysis of a solid that is precipitated from a fluid sample. This is achieved by feeding fluid sample and a precipitant to a precipitation zone. The fluid sample is added to the sample plate through an inlet, which preferably directs the sample fluid directly to the precipitation zone to reduce sample loss and improve the accuracy of the analysis.

The apparatus is particularly suitable for the analysis of a complex fluid that comprises a plurality of different components. Examples of such complex fluids include crude oil, fluids or process streams derived from the refining of crude oil, and fluids or process streams derived from Fischer-Tropsch processes. In a preferred embodiment of the invention, the apparatus is used in the determination of the asphaltenes content of crude oil, or a high-boiling product stream resulting from crude oil fractionation, for example vacuum gas oil or vacuum residue.

The precipitation zone is suitably adapted to allow mixing or other forms of agitation of the contents to ensure efficient precipitation. Thus, the precipitation zone can comprise a stirrer, suitably a magnetic stirrer, which can be controlled by a driving mechanism located in the base portion of the portable apparatus. As magnetic stirrers do not need to be physically connected to the stirrer motor, they reduce the complexity of the sample plate design and avoid problems associated with sealing any stirring equipment that would otherwise need to penetrate through the wall of the precipitation zone.

The shape and dimensions of the precipitation zone can also be adapted to optimise precipitation efficiency. The precipitation zone is preferably in the form of a chamber with a higher cross-sectional area than any transfer channels, such as microfluidic channels, that are connected thereto. This ensures more efficient precipitation and mixing with viscous liquids such as crude oil or heavy crude oil fractions, and also has a reduced tendency to block when precipitation occurs. To ensure all the solution within the precipitation zone after the precipitate has dissolved is removed from the precipitation zone and fed to the analysis zone, the precipitation zone is advantageously tapered at its outlet end. The chamber is also preferably adapted to accommodate a magnetic stirrer bar that is able to rotate freely therein. In this embodiment, the cross section of the chamber is preferably circular so as to minimise unstirred regions and improve mixing efficiency. Additionally, it is preferred that the precipitant and sample fluid are fed separately to the precipitation zone, as pre-mixing could result in the aforementioned problem of blocking due to precipitation within any of the transfer channels.

In one embodiment of the sample plate of the present invention, the inlet is adapted to allow a sampler to be introduced therein, which sampler can be inserted directly into the precipitation zone, which enables the sample fluid to be dispensed directly therein. The precipitation zone is preferably pre-loaded with precipitant before the addition of the sample in order to improve the efficiency and extent of precipitation.

The precipitate produced in the precipitation zone is separated using a filter. Further precipitant can be fed into the precipitation zone and/or through the filter after the initial filtration in order to ensure complete precipitation, and also to ensure that as much of the solid as possible is collected on the filter. The additional precipitant can also be used to wash the filtered solid of residual non-precipitated sample fluid.

Filters that can be used in the present invention can comprise fibrous or granular materials. Thus, filter paper (comprising cellulosic fibres), glass wool (comprising glass fibres), or a sintered glass frit are suitable. The filter can be located in a channel that intersects with the mixing zone, the portion of the channel housing the filter being optionally enlarged to provide a high filtration surface area. Alternatively, the filter can be associated with the precipitation zone, for example being located within the precipitation zone, or at one end thereof.

The filtered solid is re-dissolved by feeding a solvent to the filter. The solvent can be fed into the same region of the precipitation zone as the precipitant and sample fluid. Alternatively, and preferably, the solvent can be introduced from the opposite side of the filter to the solid which improves dissolution of the solid. An additional advantage of this embodiment is the solvent can be passed through the analysis zone, and background measurements taken, as it is fed to the precipitation zone, thus reducing analysis time and ensuring that there is no contamination of the analysis zone by the precipitant so that an accurate background reading can be made. The solvent then passes into the precipitation zone, where it dissolves the precipitate to form a solution. The resulting solution is then fed back though the analysis zone where the sample measurements are made.

Analysis of the solution is carried out in the analysis zone in order to determine, for example, identification of components present in the solid precipitate, and/or the concentration of solid precipitate in the solution. To this end, the analysis zone may comprise a sensor or device for detecting properties of the solution and producing an electrical signal in response thereto for transmission to related analytical apparatus, located for example in the base portion of the portable apparatus. Alternatively, the analysis zone may be adapted so that a measurement can be made on the solution therein using sensors or devices not located on the sample plate, but are instead associated with the base portion of the portable apparatus, for example.

In one embodiment of the invention, the analysis zone is in the form of an optical cell adapted to allow one or more wavelengths of electromagnetic radiation (EMR) to be directed therein, and to allow reflected and/or transmitted radiation to emanate therefrom. This can be achieved by providing the analysis zone with windows that are transparent to the EMR wavelengths used to an extent sufficient enough to allow absorbance and/or reflectance measurements to be obtained. Glass windows can be used for visible EMR wavelengths, for example. The optical analysis device itself, comprising an EMR emitter and an EMR sensor, can be located either on the sample plate or in the base portion. Wavelength regions suitable for use in the optical analysis include ultraviolet (UV), visible and infrared, including mid-infrared (MIR) and near infrared (NIR) wavelengths. Absorption and spectroscopic analysis can yield information regarding the identity and concentrations of the components therein.

Other analytical devices that can be used include a micro-oscillator device (such as a micro acoustic or acousto-optical device), a thermal conductivity device, a microconductivity or capacitance device, a micro-rheological device, or a micro-gas chromatographic (GC) device. Such devices, in combination with suitable sensors where appropriate, can be used to measure parameters such as viscosity, cold-flow properties, cloud point, density, acidity, composition, component concentrations and rheological properties.

In one embodiment of the invention, the sample plate comprises more than one analysis zone, with more than one associated analytical device, such that a plurality of measurements can be made on the solution derived from the fluid sample. In a further embodiment of the invention, the fluid sample can itself be analysed, either before or after precipitation, so that comparative analysis can be obtained.

The portable apparatus can be adapted with reservoirs for storing the precipitant, solvent or optionally any other fluid required for any treatment or analysis on the sample plate, such as any waste fluids generated during the analysis. In one embodiment, the reservoirs can be located on the sample plate, such that a sample plate comes pre-loaded with all the necessary reagents needed for the analysis, and holds any waste. Alternatively, the reservoirs can be located in the form of suitable containers located within the base portion of the apparatus, such that the solvent and precipitant reservoirs (for example) can be refilled as necessary for subsequent analysis, or changed for performing a different analysis. Additionally, the waste reservoir can be emptied when full and reused.

Alternatively, the reservoirs may not be associated with the portable apparatus, for example being stored in containers or bottles separate to the apparatus, such that fluids are pumped to or from the apparatus without the need for their storage therein. This reduces the size and complexity of the sample plate and/or base portion accordingly.

The sample plate can be adapted to receive a portion of sample fluid into a storage reservoir before the analysis. In an alternative embodiment, the sample plate is adapted to receive a sampler or sampling device which contains a pre-determined volume of sample fluid for analysis. In a further embodiment, the sampler can be inserted directly into the precipitation zone, where it can be mixed directly with precipitant.

The portable apparatus can also be provided with suitable pumping and measuring means, and also microvalves to control the paths that the fluids take to the various regions of the sample plate when in use. The micropumps and microvalves are typically associated with driving and actuating apparatus located in the base portion of the apparatus. The pumps themselves can be located on the sample plate, or alternatively the sample plate can be adapted to engage with the micropumps located together with the actuating means in the base portion. Suitable micro-pumps include diaphragm pumps, peristaltic pumps, rotary pumps, gear pumps and piston pumps. The apparatus can additionally be adapted to pump air around the various channels and zones of the sample plate for transferring any liquids around the plate, and also to flush relevant transfer lines and/or either or both of the precipitation or analysis zones. Pumps can be adapted to measure the volumes of fluids so that accurate quantitative information can be obtained. Peristaltic pumps are particularly suitable in this regard for pumping liquids.

Waste resulting from the analysis can be collected for later disposal. Waste streams include the filtrate of the fluid sample after precipitation and filtration, precipitate washings using additional precipitant, and the precipitate solution after analysis. The waste storage can be associated with the sample plate, for example in the form of a waste reservoir or chamber. In an alternative embodiment, the base portion of the portable apparatus can house a receptacle or other storage area to which the waste is directed. In a further embodiment, waste is directed to a waste receptacle separate from the portable apparatus.

The portable apparatus is suitably adapted so that processing of the relevant analytical and volumetric information can be achieved, and results in the form of a electronic or printed output can be provided. This is suitably achieved with a microprocessor that can run suitable programs to ensure the correct volumes of fluids are pumped around the sample plate, and in the correct sequence. In one embodiment, the microprocessor is programmable so that existing analysis, pumping and sequence programmes can be improved, or different analyses for different samples can be performed.

Optionally, the portable apparatus can be adapted so that different portions of the sample plate can be heated or cooled using suitable heating or cooling devices. These can also be linked with the processor so that they can be turned on or off as necessary during the analysis procedure. For example, the sample plate can comprise one or more heating devices in the form of resistively heated wires or elements associated with the portions of the sample plate to be heated. In such an embodiment, they are connected to a power supply typically located in the base portion of the apparatus. In a further embodiment, the heating devices are located in the base portion, the sample plate being adapted to accommodate them.

The portable apparatus can comprise, or at least be compatible with, wireless communications, such as a wireless mesh network, and also with remote communications means, such as satellite-based data communication, such that the analysis results may be readily communicated to the potential purchaser, again reducing the time-scale on which the analysis data is available to the potential purchaser.

The use of a portable apparatus according to the present invention requires only a small quantity of liquid sample, typically less than 100ml, such as 10 ml or less, and preferably 1ml or less. Because of the small quantity of sample required the analysis can be performed in a significantly shorter time than conventional analysis.

The sample plate of the portable apparatus is preferably replaceable, in that it can be easily attached and detached from the base portion of the portable analysis apparatus. Attachment means are suitably provided, such as click-fit type connectors or slot in type connectors, using guide rails for example.

The sample plate can be cleaned before a subsequent use. Alternatively, the sample plate can be disposed of, which can be advantageous where the reagents or sample are difficult to clean. Parts of the portable apparatus that come into direct contact with sample or other fluids such as precipitant or solvent are preferably located on the sample plate, while complex, expensive, and other devices or features that are difficult to replace are preferably located within the base portion of the portable apparatus. Thus, analytical devices such as EMR sensors and emitters, actuating mechanisms for pumps and valves, micro-processors for controlling the sequence of actuation and analysis, and so on are preferably located in the base portion of the portable apparatus. This ensures that reusable parts of the equipment do not suffer damage through cleaning or contact with sample, for example. Additionally, where disposable sample plates are used, costs associated with the portable apparatus can be minimised.

The sample plate is suitably made from ceramic, metal, glass, polymeric materials, or a combination of two or more thereof. Polymeric materials which can be injection moulded are particularly suitable. Preferably, the sample plate is resistant to leaching or other degradative effects caused by the action of the sample fluid, solvent, precipitant, or any other fluids used in the analysis. For the analysis of crude oil or products of refinery processes, suitable polymeric materials include polytetrafluoroethylene (PTFE), polyphenylene sulphide (PPS) and polyetheretherketone (PEEK).

The various components and devices of the portable apparatus are suitably microfabricated, in which techniques such as micro-moulding, electrodischarge machining, or laser micro-machining are employed in their fabrication, for example in cutting or etching microfluidic channels in the sample plate, and in the manufacture of microvalves or micropumps. Microfabricated components also include those manufactured by techniques employed in the micro-chip industry, for example sensors and micro-processors A sensor is typically used to produce an electrical signal in response to a stimulus, such as contact with a sample fluid, the solution of precipitate, or when exposed to EMR. This electrical signal is fed to an associated set of electronics which converts the input signal into a value for the property being measured.

Because of the relatively small size of the components of the portable analysis apparatus of the present invention, the power requirements are also relatively low. Hence, the portable analysis apparatus may be operated from a suitable battery (or battery pack), preferably a rechargeable battery, without the battery requirements being too heavy to impact the portability of the apparatus.

A typical process involves feeding the sample fluid and precipitant to the precipitation zone. For asphaltenes analysis of crude oil or a heavy fraction of crude oil, heptane can be used as the precipitant. The volume ratio of precipitant to the sample fluid is typically in the range of from 1 : 1 to 100 : 1. When using heptane as a precipitant for asphaltenes, the precipitant to sample fluid volume ratio is suitably in the range of from 5 : 1 to 50: 1.

The mixture of sample fluid and precipitant can be held in the precipitation zone, preferably with agitation from a stirrer for example, to improve the extent and efficiency of precipitation. This is typically in the range of from 1 to 60 minutes before filtration. For crude oil and heptane mixtures, efficient asphaltene precipitation is typically achieved within a period ranging from 3 to 15 minutes, the precipitation time preferably being at least 5 minutes for improved accuracy.

The suspension formed in the precipitation zone is passed through the filter to separate the precipitated solid. Further precipitant can additionally be fed into the precipitation zone and through the filter to remove residual fluid sample.

A solvent is then fed to the filter, and also preferably into the precipitation zone, to dissolve the solid. The solvent is chosen so that the solubility of the precipitated solid is sufficiently high so as to dissolve the solid rapidly and efficiently. However, it should also be chosen so that it does not interfere in any subsequent analysis of the dissolved solid. For example, if a spectroscopic analysis is to be carried out, the solvent should not strongly absorb at wavelengths in the same regions of the spectrum as the precipitate.

For the analysis of asphaltenes from crude oil or refinery products, suitable solvents include dichloromethane (DCM), acetone and toluene. DCM is preferred, as it does not absorb EMR in the region of the UV/Visible spectrum where asphaltenes are highly absorbing.

Determining the concentration of asphaltenes in a crude oil sample provides useful information as to how the crude will behave when fed to a refinery, for example in helping to predict how much heavy residue will be produced, or the potential extent of fouling of process equipment or any catalysts used in refinery processes downstream of a crude distillation unit.

The portable apparatus can be adapted to allow other analyses to be carried out, either on the solution or on the sample fluid either before or after precipitation and filtration so that comparative analysis can be performed. The sample plate can also have more than one analysis zone, allowing the one or more fluids to be analysed by more than one analytical technique. Thus, the process of the present invention can allow analysis of the sample fluid before and/or after filtration by one or more analysis techniques in one or more analysis zones.

Parameters calculated from the analytical results obtained by the process of the present invention may not necessarily be obtained directly. For example, a number of parameters can be predicted or calculated from other measurements. In one embodiment, this is achieved using chemometrics techniques, in which the parameters measured are used to calculated non-measured parameters by applying a predictive model, which is typically derived from database of previous analytical measurements. Typical chemometrics techniques include partial least squares analysis, and principal component regression.

Because of the rapid analysis obtainable from the portable apparatus of the present invention, results obtained therefrom can be used in process optimisation. For example, the portable apparatus may be used at a refinery for performing regular analyses on blends of crude oils, produced when crude oils from different sources are present in a single storage tank. This allows the configuration or operating conditions in one or more of the refinery processes to be planned in advance as a result of the analysis. Furthermore, the portable apparatus may be used to verify consistency and/or quality of feedstocks on arrival at a refinery or blending station, or on process streams within the refinery, such that feedstock quality and property data can be obtained and input into blending and process refinery optimisation models.

There now follows non-limiting examples of sample plates and portable analytical apparatus according to the present invention. Also illustrated is a method of determining the asphaltene concentration of a crude oil sample using a sample plate and portable apparatus according to the present invention. The invention is illustrated with reference to the Figures, in which:
Figure 1 is a schematic illustration of the features of a sample plate and base portion of a portable analysis apparatus according to the present invention.
Figure 2 is an exploded view of two sub-plates of a sample plate according to the present invention, the face shown being the face that interengages with a base portion in order to form a portable analysis device.
Figure 3 is a view of the portion of the precipitation zone on one of the sub plates of the sample plate of Figure 2, showing how the precipitation zone tapers at one end.
Figures 4 is an overhead view of the same face of the sample plate as shown in Figure 2, with the sub-plates engaged with each other.
Figures 5 is a view of the opposite face of the sample plate shown in Figure 4.
Figure 6 is a cutaway view of the sample plate of Figures 2 to 5, showing a microvalve.
Figure 7 is a perspective view of the face of a base portion of a portable analysis apparatus, which engages with the face of the sample plate as shown in Figures 2 and 4.
Figure 8 is a longitudinal section through an optical analysis device associated with the base portion of Figure 7.
Figure 9 illustrates the sample plate of Figures 2 to 5 when engaged with the base portion of Figure 7.
Figure 10 is a graph showing the relationship between absorbance at 560nm and 640nm versus asphaltene concentration in DCM solutions.
Figure 11 is a perspective view of a sampler in the open position that can be used with the sample plate shown in Figures 2 to 5.
Figure 12 is a perspective view of the sampler of Figure 9 in the closed position.
Figure 13 illustrates the opening, closing and locking mechanism of the sampler of Figures 11 and 12.

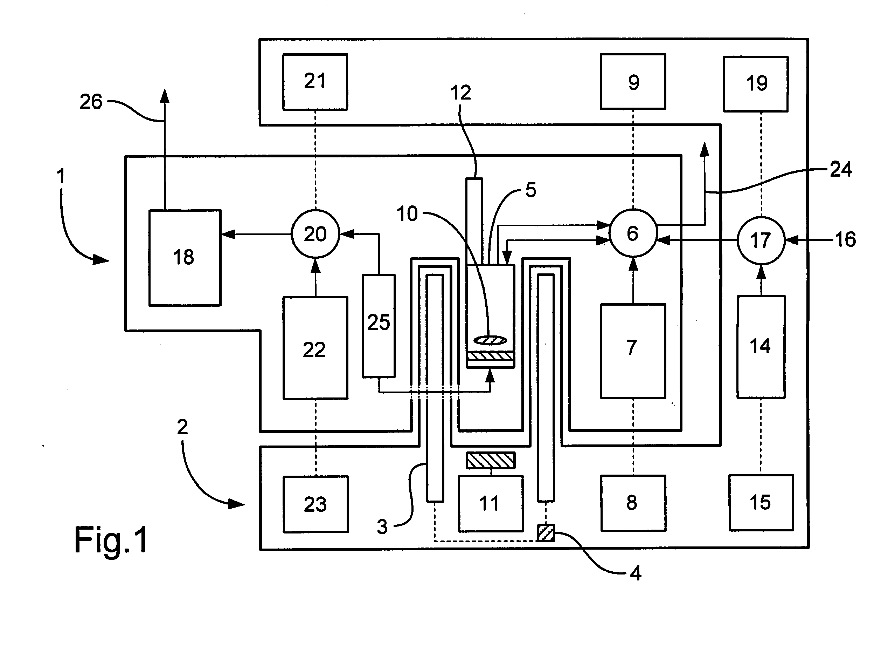

Figure 1 schematically illustrates a sample plate 1 associated with a base portion 2 of a portable apparatus that can be used for the determination of the asphaltene concentration of a sample of crude oil. In use, a user attaches the sample plate 1 to the base portion 2, and switches on a heater comprising resistively heated elements 3 controlled by temperature controller 4. The heating elements heat the precipitation zone 5 to a temperature of 40°C. Higher temperatures can be used if desired. 2 ml n-heptane precipitant are pumped to the precipitation zone 5 through valve 6 from a reservoir 7 using a piston pump, driven by a stepper motor 8. The valve position is changed using a geared valve motor 9. Magnetic stirrer 10, controlled by a stepper motor 11, is switched on and the heptane is stirred until the temperature reaches 40°C, which is achieved within 2 minutes. A sampler 12, which in this embodiment is a separate apparatus that can be added to or removed from the sample plate, is filled with crude oil and inserted into an appropriate inlet on the sample plate. The sampler is opened by the user, and crude oil is discharged into the precipitation zone, where it mixes and is stirred with the heptane for a period of 15 minutes.

Precipitated asphaltenes are collected on filter 13 by switching valves 6, 17 and 20 using valve motors 9, 19 and 21 respectively, and using a piston driven air pump 14, controlled by stepper motor 15, to pump air 16 through valves 17 and 6, to force the suspension in the precipitation zone through the filter, wherein the filtrate is collected in waste reservoir 18 fitted with vent 26. Precipitated asphaltenes are washed by switching valve 6 accordingly, and pumping a further 2 ml n-heptane from reservoir 7. The heptane is held in contact with the precipitated asphaltenes for 2 minutes. Once the heptane has been added, valve 6 is returned to its previous position, and air pump 14 is used to push air through the apparatus, and force the additional heptane to waste reservoir 18.

Valve 20 is switched to allow dichloromethane (DCM) solvent to be delivered from solvent reservoir 22 to the precipitation zone 5 using a piston pump controlled by stepper motor 23. At the same time, valve 6 is switched to open vent 24, and thus allowing transfer of the solvent. As the DCM passes through an optical analysis zone 25, background visible absorption measurements are collected, using an optical analysis device (not shown) comprising an array of three LED emitters (red, green and blue light) and a photodiode detector. The optical analysis zone has windows transparent to the EMR, which allow the EMR from the emitters to pass through the optical analysis zone to the detector, while at the same time preventing leakage of any fluids therein. Absorption at red, green and blue wavelengths is carried out sequentially. The DCM is held in contact with the asphaltenes on the filter for 2 minutes. The stirrer motor 11 is then turned off, and valves 6 and 20 are switched to allow air pump 14 to drive the DCM/asphaltene solution back through the optical analysis zone 25, and through to waste reservoir 18. As the solution passes through the optical analysis zone, visible absorption measurements of the solution are collected. Vents 24 and 26 can optionally be fitted with an absorbent, such as an activated carbon absorbent, in order to reduce the extent of vapours from the fluids being emitted to the atmosphere. The complete sequence takes approximately 40 minutes.

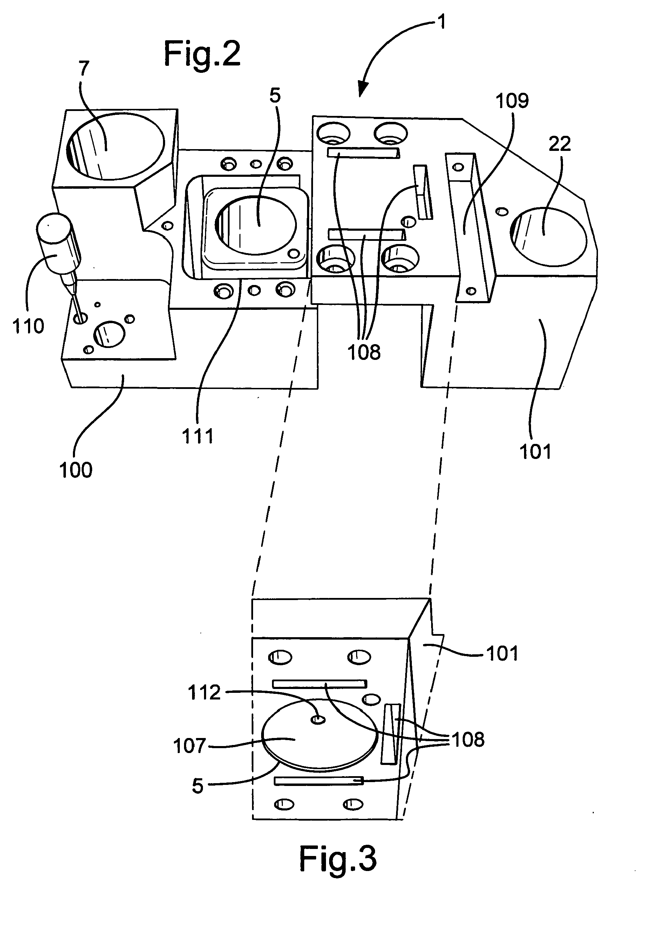

Figure 2 shows an exploded view of a sample plate 1 which can be used in the determination of asphaltenes in crude oil, as outlined in the description of Figure 1. The sample plate 1 comprises two interconnecting sub-plates 100 and 101. Further sub-plates (not shown) act to seal exposed parts of the plate that do not interface with or are not closed off by parts of the base portion of the portable analysis apparatus, for example the microfluidic channels.

On sub-plate 100 can be seen the precipitant reservoir 7 and air-pump adaptor 110. In one embodiment, the air pump is an air-filled syringe, the nose of which inserts into adaptor 110, and the plunger of which engages with a motorised syringe driver 15, as identified in Figure 1.

Also shown is the precipitation zone 5, surrounded by a recess 111, into which heating elements (not shown) can be inserted for controlling the temperature within the precipitation zone.

On the second sub-plate 101 is shown the solvent reservoir 22, and a recess 109 which accommodates an optical analysis device (not shown). The aforementioned heating elements from the base portion of the portable apparatus (not shown) are inserted into recess 111 through slits 108.

Figure 3 in an enlarged view of the opposite face of the portion of the precipitation zone 5 associated with sub-plate 101, as indicated by the dashed arrows. The tapered end 107 of the precipitation zone 5 is visible, as is the outlet of the precipitation zone 112 that leads to analysis zone 25 (not shown).

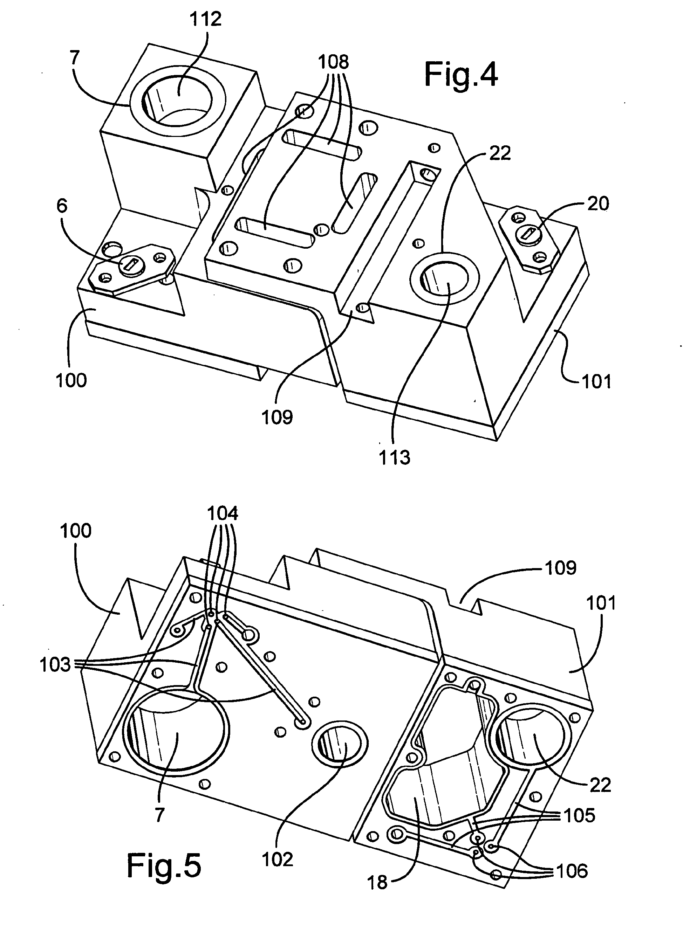

Figure 4 shows the two sub-plates of the sample plate of Figure 2 when engaged. Further features shown include microvalve 6 which controls the path of air from the air pump, of precipitant from the reservoir 7, and of precipitation zone contents 5 to and from the microvalve. Microvalve 20 is also shown, which directs the passage of fluids between the solvent reservoir 22, the waste reservoir (not shown) the analysis zone, located in recess 109.

The solvent 22 and precipitant 7 reservoirs can be adapted to host pistons 112 and 113 respectively from associated piston pumps 8 and 23 (not shown).

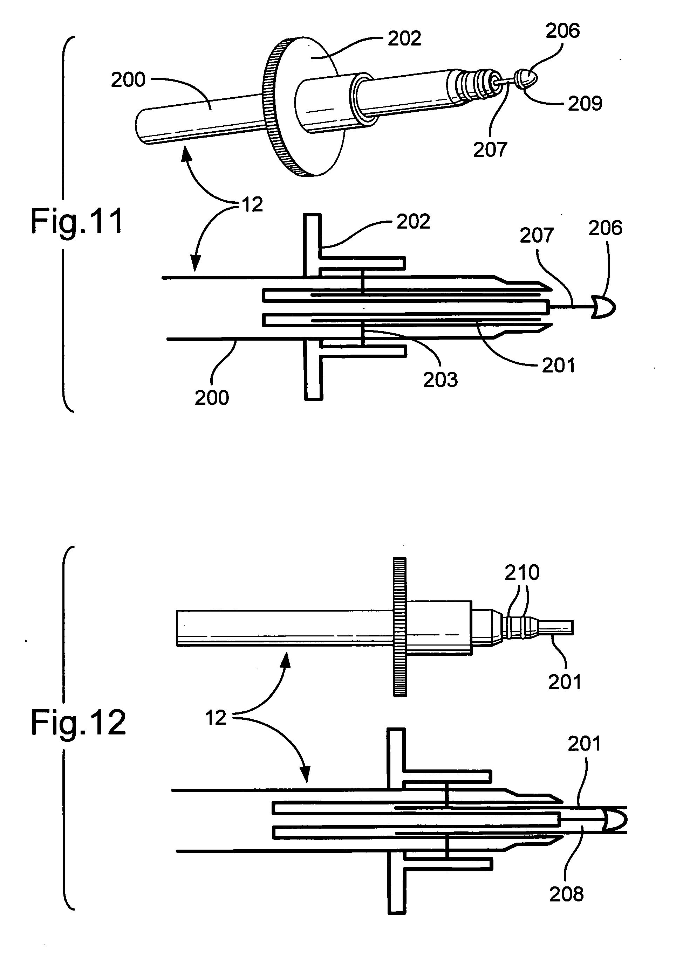

Figure 5 shows the opposite face of the sample plate of Figure 4. On sub-plate 100 can be seen the precipitant reservoir 7 and the sample inlet 102, together with a number of microfluidic channels 103. Channels 103 intersect with microvalve 6 (not shown) at a series of microvalve inlets 104. The microfluidic channels 103 provide pathways for fluids between the microvalve and the air pump via adapter 110 (not shown), the precipitant reservoir 7, the precipitation zone 5, and vent 24 (not shown). The sample inlet leads into the precipitation zone 5 (not shown), and allows the sample-containing head of a sampler, as shown in Figures 11 to 13, to be inserted directly into the precipitation zone for dispensation of the sample fluid to be analysed. The width of the microchannels 103 and 105 is 2mm.

On sub-plate 101, solvent reservoir 22 and a waste reservoir 18 are shown, together with a number of microfluidic channels 105 that intersect with microvalve 20 (not shown) as a series of microvalve inlets 106. The microchannels provide pathways for fluids between the the microvalve and solvent reservoir 22, the waste reservoir 18, and the analysis zone located in recess 109.

The sample plate illustrated in Figures 2 to 5 comprises a plurality of sub-units that fit together to form a single plate. However, a sample plate consisting of just a single unit is also within the scope of the present invention.

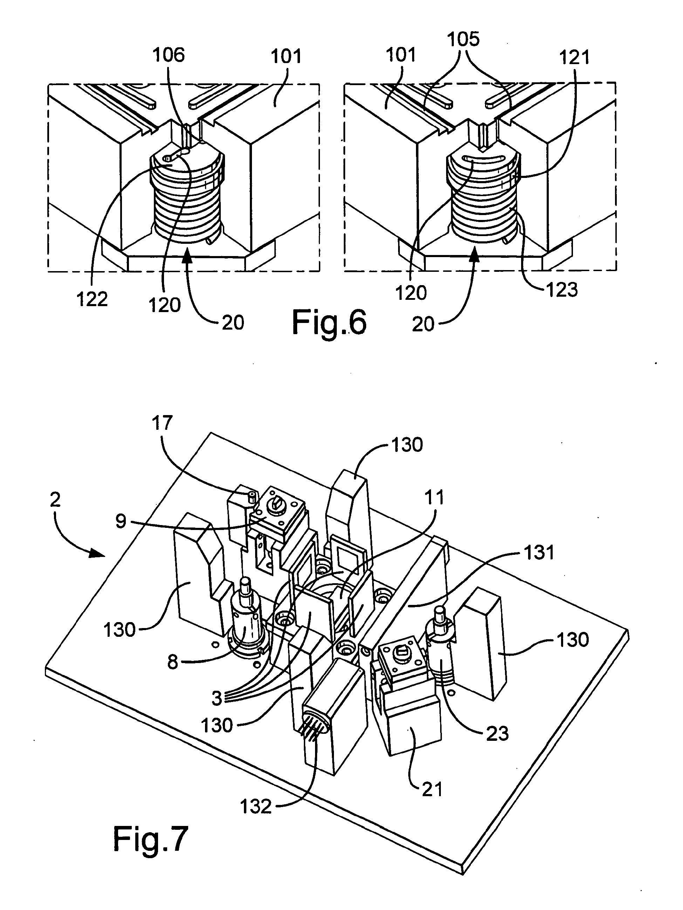

Figure 6 is a cut-away view of the sub-plate 101, showing microvalve 20 associated with the microvalve inlets 106 of the microfluidic channels 105. The microvalve 20 comprises a microvalve head 121, with a layer of sealing material 122. The layer of sealing material comprises channels or grooves 120, which define passages between two microvalve inlets, and hence between the associated microfluidic channels 105, depending on the position of the microvalve. The microvalve is controlled by an actuator, for example a geared or stepper motor, located within the base portion of the portable apparatus, which rotates the valve. The sealing layer 122 on the microvalve head prevents leakage of fluids from the sample plate into the other microfluidic channels that are not connected by the grooves 120. The microvalve can also be provided with a spring 123, which pushes the head of the microvalve against the sample plate in order to improve the sealing effect. Tests show that such a valve can be leak tight up to a fluid pressure of about 7 barg (0.8MPa).

Figure 7 shows features of a base portion 2 of a portable apparatus that engages with a sample plate as illustrated in Figures 2 to 5. Guide rails 130 insert into appropriate cavities in the sample plate (not shown), and help ensure that the relevant portions of the sample plate interface appropriately with the devices in the base portion. Stepper motors 9 and 21 are used to drive the microvalves 6 and 20. Air pump 17 connects to the adaptor 110 of the sample plate. Pump motors 8 and 23 drive pistons to deliver, respectively, precipitant and solvent into the relevant portions of the sample plate. Heater elements 3 surround the precipitation zone of the sample plate, passing through the slits 108 of the second sub-plate 101, and into the recess 111 (not shown) when the sample plate engages with the base portion. Magnetic stirrer motor 11, positioned between the four heating elements 3, operates a magnetic stirrer bar 10 (not shown) which is located in the precipitation zone when the portable apparatus is in use. Optical analysis device 131, fits in the recess 109 sub-plate 101. The optical analysis device 131 is fitted with an optical sensor 132, which converts EMR hitting the sensor into an electrical signal, which is transmitted to associated electrical apparatus located within the base portion of the portable analysis apparatus.

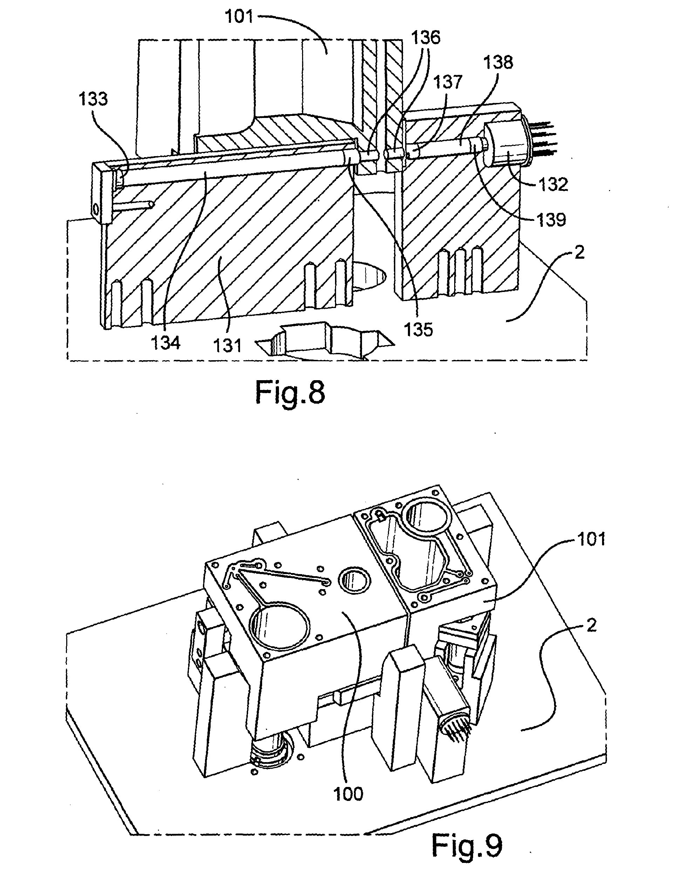

Figure 8 shows a longitudinal section through optical analysis device 131 on the base portion 2, which engages with the sample plate at channel 109. The device comprises an array of three LED emitters 133, which direct three EMR wavelengths in the red, blue and green regions of the visible spectrum. The EMR is transmitted along a polymethylmethacrylate (PMMA) optical fibre 134, and through a lens 135. The sub-plate 101, in channel 109, has two glass windows 136 which allow transmission of the EMR through the analysis zone. The EMR transmitted through and/or reflected from the contents of the analysis zone pass through a second lens 137, along a second PMMA fibre 138, and through a third lens 139, before reaching the optical sensor 132, which produces electrical signals in response to the transmitted and/or reflected EMR.

Figure 9 illustrates the sample plate when attached to the base portion of the portable apparatus, showing the interrelationship between the features of the base portion 2 and the two sub-units of the sample plate 100 and 101.

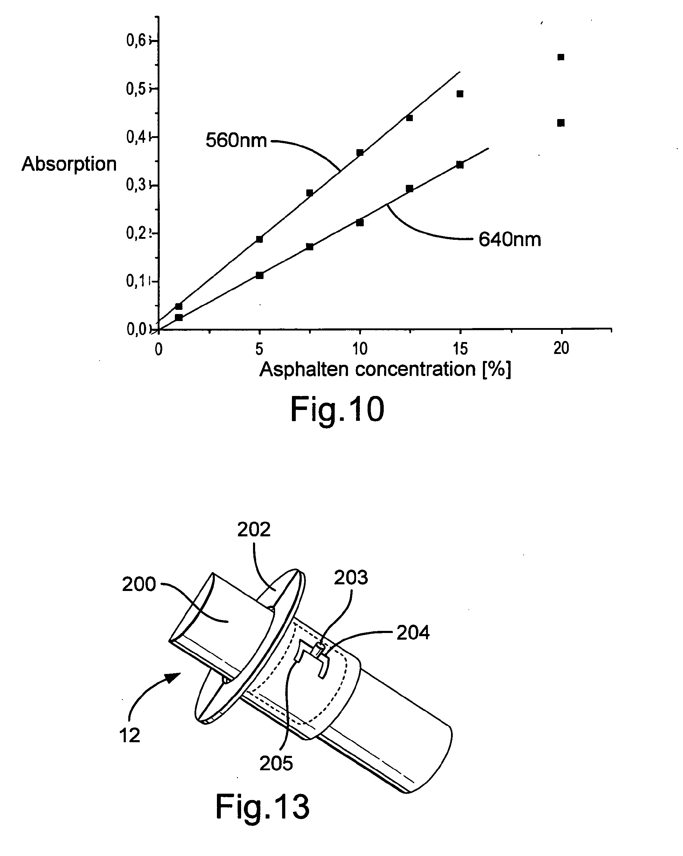

Using the method and apparatus as detailed above, it was found that wavelengths in the range of from 400 to 700 nm were optimal for determining asphaltene concentrations of up to 15% by weight. For asphaltene concentrations of less than about 7.5 wt%, wavelengths below 600 nm give better accuracy due to the higher gradient of the absorbance versus concentration relationship. However, at higher concentrations, the gradient deviates from linear, and measurements at wavelengths of 600nm or above provide greater accuracy. The relationship between asphaltene concentration and absorbance at two different wavelengths of 560nm and 640nm is illustrated in Figure 10.

A sampler used for collecting a pre-determined volume of sample fluid and dispensing it into the precipitation zone of the sample plate is illustrated in Figures 11, 12 and 13. Figure 11 shows the sampler in the open position, and Figure 12 in the closed position. The sampler is suitable for accurately collecting and dispensing low sample volumes, particularly of 0.5 mL or less, and can be used accurately to dispense samples of 0.1 mL or less. The dispenser is particularly useful for the accurately dispensing measured volumes of viscous samples such as crude oil. For the embodiment shown in Figure 9, samplers that accurately dispense crude oil volumes of 50 and 100µl have been made.

In general, the sampler comprises a first and second member, arranged so that they can slide relative to one another. The members are adapted and inter-engaged to provide a sample cavity that can be opened and closed as a result of the relative sliding movement of the two members, allowing collection, entrapment and discharge of a sample fluid. In one embodiment, the sliding movement is achieved by attaching one of the members to a handle, as shown in Figure 13 which shows a cut-away portion of the handle showing the connection to the sliding member.

In the embodiment shown in Figure 13, the sampler 12 comprises a housing 200 enclosing a hollow shank 201 (not shown). The hollow shank is connected to a handle 202 by struts 203, which extend through channels 204 in the housing, which channels allow longitudinal movement of the shank relative to the housing on moving the handle. The channels can be adapted to allow the handle to be locked in place, which is suitably achieved by providing transverse extensions 205 to the channels that, when aligned with the struts, allow rotational movement of the shank 201 in order to lock sampler in an open or closed position.

The housing comprises a head 206 connected to the remaining body of the housing by a neck 207, which is narrower than the body and the head. When the sampler is in the closed position, the hollow shank 201 extends down through the housing 200 covering the narrow neck 207 portion and contacts the head 206 to form a sample cavity 208. In the open position, the shank does not engage with the head, and in one embodiment is fully retracted into the body of the housing.

In use, the sampler is opened and placed into the sample fluid to be analysed. The sampler is then closed by pushing the handle down and entrapping sample fluid within the sample cavity. The sampler is then removed from the sample fluid, wiped clean, and transferred to the sample plate, where it can be inserted into the appropriate sampler inlet 102. The sample is released from the cavity by pulling up the handle, uncovering the sample cavity, and allowing the sample fluid to be dispensed.

The distance of the head from the body of the housing, and the dimensions of the neck that connects them, are typically chosen so that the neck can support the head without being easily broken, while at the same time ensuring that the desired volume of sample fluid can be enclosed within the sample cavity. Having an elongated arrangement, i.e. a long, narrow sample cavity as opposed to a short and wide sample cavity, improves the accuracy and reproducibility of the volumes of sample fluid that can be captured and dispensed.

The shank can extend over at least part of the head of the housing. The head can optionally be adapted with a seal 209 that prevents leakage between the shank and the head. Additionally, the shank can optionally comprise one or more seals to prevent sample leaking from the source of sample fluid or from the cavity and into the space between the shank and the body of the housing. The housing can also be adapted with seals 210 so that, when in use, contents of the sample plate do not leak through the sampler inlet of the sample plate during analysis.

## Claims

1. A sample plate for a portable analysis apparatus for the analysis of a solid precipitated from a fluid sample, which sample plate comprises a sample inlet, a precipitation zone, a filter and an analysis zone, the sample plate being adapted to allow:
(i) a fluid sample to be fed through the sample inlet into the precipitation zone;
(ii) a precipitant to be fed to the precipitation zone;
(iii) conditions within the precipitation zone to be maintained such that precipitation occurs when the fluid sample and the precipitant mix to form a suspension;
(iv) separation of the solid in the suspension by the filter;
(v) addition of a solvent to the filter to dissolve the solid and form a solution; and
(vi) analysis of the solution in the analysis zone.

2. A sample plate as claimed in claim 1, comprising one or more microfluidic channels.

3. A sample plate as claimed in claim 2 in which the precipitation zone is in the form of a chamber with a higher cross-sectional area than microfluidic channels connected thereto.

4. A sample plate as claimed in any one of claims 1 to 3 additionally comprising one or more microvalves.

5. A sample plate as claimed in any one of claims 1 to 4, in which the precipitation zone is tapered at its outlet end.

6. A sample plate as claimed in any one of claims 1 to 5, in which the precipitation zone comprises a magnetic stirrer.

7. A sample plate as claimed in any one of claims 1 to 6, in which the filter is within the precipitation zone.

8. A sample plate as claimed in any one of claims 1 to 7, having an inlet for air.

9. A sample plate as claimed in any one of claims 1 to 8, having one or more vents.

10. A sample plate as claimed in claim 9, in which the vents have an absorbent.

11. A sample plate as claimed in any one of claims 1 to 10, in which the analysis zone is an optical analysis zone.

12. A sample plate as claimed in claim 11 in which the optical analysis zone comprises one or more windows that are transparent to the EMR wavelengths used.

13. A sample plate as claimed in claim 12, in which the windows are glass windows.

14. A sample plate as claimed in any one of claims 1 to 13 additionally comprising one or more of a waste reservoir, a solvent reservoir, a precipitant reservoir and a reservoir for the sample fluid.

15. A sample plate as claimed in any one of claims 1 to 14, fabricated from metal, glass, ceramic, or polymeric materials.

16. A sample plate as claimed in claim 15, in which the sample plate is fabricated from polymeric materials selected from PEEK, PPS or PTFE.

17. A sample plate as claimed in any one of claims 1 to 16 in which the inlet is adapted to receive a sampler which contains sample fluid.

18. A portable analytical apparatus for the analysis of a solid precipitated from a fluid sample comprising a sample plate and a base portion, in which the sample plate is a sample plate according to any one of claims 1 to 17.

19. A portable analytical apparatus as claimed in claim 18 additionally comprising micropumps.

20. A portable analytical apparatus as claimed in claim 19, in which the micropumps are selected form one or more of the group comprising diaphragm pumps, peristaltic pumps, rotary pumps, gear pumps and piston pumps.

21. A portable analytical apparatus as claimed in claim 19 or claim 20 in which the base portion comprises actuators for controlling microvalves and micropumps.

22. A portable analytical apparatus as claimed in any one of claims 18 to 21 comprising heating and/or cooling devices for controlling the temperature in various portions of the sample plate.

23. A portable analytical apparatus as claimed in claim 22, in which the heating and/or cooling devices are located in the base portion.

24. A portable analytical apparatus as claimed in any one of claims 20 to 23, in which the sample plate has an optical analysis zone, and the base portion has an optical analysis device that engages with the optical analysis zone of the sample plate.

25. A portable analytical apparatus as claimed in claim 24, in which the optical analysis device comprises one or more LED emitters.

26. A portable analytical apparatus as claimed in any one of claims 18 to 25, having a microprocessor for controlling the sequence of microvalve and/or motor operations.

27. Use of a sample plate according to any one of claims 1 to 17 or a portable analytical apparatus according to any one of claims 18 to 26 for analyzing a solid precipitated from a fluid sample.

28. Use of a sample plate or portable analytical apparatus according to claim 27, in analysing asphaltenes in crude oil or a heavy fraction derived from crude oil distillation.

29. A sampler for collecting and dispensing sample fluid into a sample plate in accordance with claim 17, which sampler comprises a first and second member arranged so that they can slide relative to one another, **characterised by** one or both members being adapted to provide a sample cavity that can be opened and closed as a result of the relative sliding action of the first and second members, and which allows the collection and entrapment of fluid sample within the cavity, and discharge therefrom.

30. A sampler as claimed in claim 29, in which the first member is a housing which encloses the second member, which second member is a hollow shank, wherein the housing comprises a body and a head connected by a neck, which neck is narrower than the body and the head, and which neck forms a cavity which can be reversibly closed and uncovered by the sliding action of the hollow shank relative to the housing.

31. A sampler as claimed in claim 29 or claim 30, in which the second member connects to a handle.

32. A sampler as claimed in claim 31, in which the handle connects to the second member through one or more channels in the first member that allow longitudinal relative movement between the first and second members.

33. A sampler as claimed in claim 32, in which the channels are adapted to allow the handle to be locked in place.

34. A sampler as claimed in claim 33, in which the channels have transverse extensions.

35. A sampler as claimed in any one of claims 29 to 34, having one or more seals for preventing leakage of sample fluid.
